# FASCICULE DE BREVET EUROPEEN

(11) **EP 0 918 506 B1**
(45) Date de publication et mention de la délivrance du brevet: **22.08.2001**
(21) Numéro de dépôt: 97934602.0
(22) Date de dépôt: 21.07.1997
(51) Int. Cl.: A61K 7/48

(54) **COMPOSITIONS LAVANTES ET CONDITIONNANTES A BASE DE SILICONE ET DE DIALKYLETHER**
KONDITIONIERENDE UND WASCHENDE MITTEL AUF BASIS VON SILIKON UND DIALKYLETHER
WASHING AND CONDITIONING COMPOSITIONS CONTAINING SILICONE AND DIALKYLETHER

(30) Priorité: 23.07.1996 FR 9609252
(43) Date de publication de la demande: 02.06.1999
(73) Titulaire: L'OREAL, 75008 Paris (FR)
(72) Inventeur: SEBAG, Henri, F-75016 Paris (FR); DECOSTER, Sandrine, F-93800 Epinay sur Seine (FR)
(74) Mandataire: Le Blainvaux Bellegarde, Françoise
(86) Numéro de dépôt international: FR9701350
(87) Numéro de publication internationale: WO9803155

(56) Documents cités:
- EP-A- 0 264 844
- WO-A-94/16668
- FR-A- 2 134 451
- CHEMICAL ABSTRACTS, vol. 79, no. 16, 22 octobre 1973 Columbus, Ohio, US; abstract no. 96851h, MAMORU SUZUKI ET AL: "Cosmetic preparations containing higher ethers" page 317; XP002030634 & JP 07 333 037 A (POLA CHEMICAL INDUSTRY) 7 mai 1973

## Description

La présente invention est relative à des compositions moussantes de lavage et de conditionnement des matières kératiniques, en particulier des cheveux et/ou de la peau, à base de silicone, d'agent tensio-actif et d'un dialkyléther gras qui est solide à une température inférieure ou égale à environ 30°C, le pouvoir moussant de la composition étant supérieur à 50 ml, ainsi qu'aux procédés de lavage et de conditionnement mettant en oeuvre ces compositions.

Les compositions de lavage des matières kératiniques, notamment les shampooings, sont bien connues dans l'état de la technique. On a déjà proposé dans le passé d'utiliser dans de telles compositions, des silicones (agents de conditionnement) afin de conférer aux matières traitées, notamment aux cheveux, de bonnes propriétés cosmétiques telles que la douceur, la brillance et la facilité de démêlage.

Compte tenu du caractère insoluble des silicones utilisables dans les compositions moussantes de lavage et de conditionnement, on cherche à maintenir les silicones en dispersion régulière dans le milieu sans cependant faire chuter la viscosité et les propriétés détergentes et moussantes des compositions. Les silicones doivent également être véhiculées sur les matières kératiniques traitées en vue de leur conférer, suivant l'application, des propriétés de douceur, de brillance et de démêlage.

Il existe à ce jour peu de moyens pour maintenir efficacement en suspension dans des compositions de shampooing les agents de conditionnement insolubles, car il s'agit là d'un problème difficile à résoudre ; à cet effet, on a déjà proposé l'utilisation des dérivés d'esters ou d'éthers à longue chaîne (agents nacrants)(EP-A-181773 et EP-A-457688) ou des polysaccharides tels que la gomme de xanthane (gélifiants). Cependant, les agents nacrants présentent des problèmes de cristallisation qui entraînent une évolution (augmentation) de la viscosité des compositions au cours du temps ; les agents gélifiant présentent également des inconvénients, à savoir d'une part que la mousse des compositions détergentes contenant de la gomme de xanthane se développe difficilement (mauvais démarrage de mousse) et que, d'autre part, les compositions n'ont pas une texture lisse et s'écoulent par paquets, ce qui est peu apprécié des utilisateurs.

La demande EP-A-264 844 décrit des émulsions huile dans eau contenant un dialkyléther pour obtenir des émulsions liquides non moussantes et non détergentes.

La demanderesse a découvert, ce qui fait l'objet de l'invention, qu'en utilisant dans des compositions moussantes de lavage à base de silicones insolubles et d'agents tensio-actifs, au moins un dialkyléther gras solide à une température inférieure ou égale à 30°C environ, il était possible d'obtenir des compositions présentant une très bonne homogénéité et une stabilité améliorée en particulier de la viscosité tout en maintenant un pouvoir moussant suffisant. Les compositions présentent un très bon effet nacrant ainsi qu'une viscosité satisfaisante pour l'application sur les matières kératiniques. Les compositions présentent enfin une texture non filante et fondante, ce qui permet une bonne répartition de la composition sur l'ensemble de la chevelure lors de l'application.

Les compositions ainsi préparées possèdent de bonnes propriétés détergentes et moussantes.

Ces compositions, lorsqu'elles sont appliquées sur les cheveux, possèdent, en plus de leurs propriétés lavantes, des propriétés de conditionnement des cheveux, c'est-à-dire que les cheveux traités sont brillants, se démêlent facilement et sont doux au toucher.

Ces compositions, lorsqu'elles sont appliquées sur la peau confèrent aux matières kératiniques, notamment à la peau, une grande douceur.

L'invention a donc pour objet de nouvelles compositions moussantes de lavage et de conditionnement à base de silicone, d'agents tensio-actif et de dialkyléther décrit ci-après, le pouvoir moussant de la composition étant supérieur à 50 ml.

Un autre objet de l'invention est constitué par le procédé de lavage et de conditionnement mettant en oeuvre de telles compositions.

L'invention a encore pour objet l'utilisation d'un dialkyléther gras qui est solide à une température inférieure ou égale à environ 30°C comme agent de mise en suspension d'une silicone dans une composition moussante de lavage et conditionnement contenant, dans un milieu aqueux cosmétiquement acceptable, des tensioactifs.

D'autres objets de l'invention apparaîtront à la lecture de la description et des exemples qui suivent.

Le pouvoir moussant de la composition est mesurée selon la méthode Ross Miles (NF T 73-404 et ISO696) modifiée.
Les modifications de la méthode sont les suivantes :
La mesure se fait à la température de 22°C avec de l'eau osmosée. La concentration de la solution est de 2g/l. La hauteur de la chute est de 1m. La quantité de composition qui chute est de 200 ml. Ces 200 ml de composition tombent dans une éprouvette ayant un diamètre de 50 mm et contenant 50 ml de la composition à tester. La mesure est faite 5 minutes après l'arrêt de l'écoulement de la composition.

Les compositions moussantes de lavage et de conditionnement des matières kératiniques, en particulier des cheveux et de la peau, conformes à l'invention comprennent, dans un milieu aqueux cosmétiquement acceptable, au moins une silicone, au moins un agent tensio-actif possédant des propriétés détergentes et au moins un diaikyléther de formule (I) ;

R-O-R' (I)

dans laquelle :
R et R', identiques ou différents, désignent un radical alkyle, saturé ou insaturé, linéaire ou ramifié, comportant de 12 à 30 atomes de carbone et de préférence de 14 à 24 atomes de carbone, R et R' étant choisis de façon telle que le composé de formule (I) soit solide à une température inférieure ou égale à 30° C environ, le pouvoir moussant de la composition étant supérieur à 50 ml.

Plus particulièrement R et R' sont identiques.

De façon préférentielle, R et R' désignent un radical stéaryle.

Les dialkyléthers utilisables selon l'invention peuvent être solubles ou insolubles dans les compositions, mais de préférence ils sont insolubles.

Ces composés peuvent être préparés selon le procédé décrit dans la demande de brevet DE 41 27 230.
Un distéaryléther utilisable dans le cadre de la présente invention, est notamment vendu sous la dénomination CUTINA KE 3178 par la société HENKEL.

Les silicones utilisables conformément à l'invention sont en particulier des polyorganosiloxanes insolubles dans la composition et peuvent se présenter sous forme d'huiles, de cires, de résines ou de gommes.

Les organopolysiloxanes sont définis plus en détail dans l'ouvrage de Walter NOLL "Chemistry and Technology of Silicones" (1968) Academie Press. Elles peuvent être volatiles ou non volatiles.

Lorsqu'elles sont volatiles, les silicones sont plus particulièrement choisies parmi celles possédant un point d'ébullition compris entre 60° C et 260° C, et plus particulièrement encore parmi :
(i) les silicones cycliques comportant de 3 à 7 atomes de silicium et de préférence 4 à 5. Il s'agit, par exemple, de l'octaméthylcyclotétrasiloxane vendu notamment sous le nom de "VOLATILE SILICONE 7207" par UNION CARBIDE ou "SILBIONE 70045 V 2" par RHONE POULENC, le décaméthylcyclopentasiloxane vendu sous le nom de "VOLATILE SILICONE 7158" par UNION CARBIDE, "SILBIONE 70045 V 5" par RHONE POULENC, ainsi que leurs mélanges.
   On peut également citer les cyclocopolymères du type diméthylsiloxanes/ méthylakylsiloxane, tel que la "SILICONE VOLATILE FZ 3109" vendue par la société UNION CARBIDE. de structure chimique: 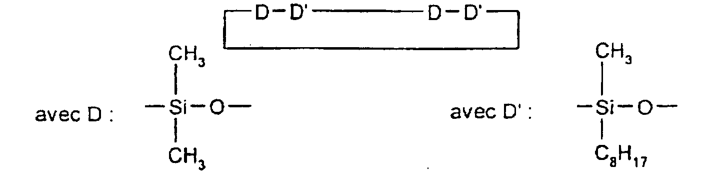
   On peut également citer les mélanges de silicones cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'(hexa-2,2,2',2',3,3'-triméthylsilyloxy) bis-néopentane ;
(ii) les silicones volatiles linéaires ayant 2 à 9 atomes de silicium et possédant une viscosité inférieure ou égale à 5.10⁻⁶m²/s à 25° C. Il s'agit, par exemple, du décaméthyltétrasiloxane vendu notamment sous la dénomination "SH 200" par la société TORAY SILICONE. Des silicones entrant dans cette classe sont également décrites dans l'article publié dans Cosmetics and toiletries, Vol. 91, Jan. 76, P. 27-32 - TODD & BYERS "Volatile Silicone fluids for cosmetics".

On utilise de préférence des silicones non volatiles et plus particulièrement des polyalkylsiloxanes, des polyarylsiloxanes, des potyalkylarylsiloxanes, des gommes et des résines de silicones, des polyorganosiloxanes modifiés par des groupements organofonctionnels ainsi que leurs mélanges.

Ces silicones sont plus particulièrement choisies parmi les polyalkylsiloxanes parmi lesquels on peut citer principalement les polydiméthylsiloxanes à groupements terminaux triméthylsilyle ayant une viscosité de 5.10⁻⁶ à 2,5 m²/s à 25°C et de préférence 1.10⁻⁵ à 1 m²/s.

Parmi ces polyalkylsiloxanes, on peut citer à titre non limitatif les produits commerciaux suivants :
- les huiles SILBIONE des séries 47 et 70 047 ou les huiles MIRASIL commercialisées par RHONE POULENC telles que par exemple l'huile 70 047 V 500 000 ;
- les huiles de la série MIRASIL commercialisées par la société RHONE POULENC ;
- les huiles de la série 200 de la société DOW CORNING ;
- les huiles VISCASIL de GENERAL ELECTRIC et certaines huiles des séries SF (SF 96, SF 18) de GENERAL ELECTRIC.

On peut également citer les polydiméthylsiloxanes à groupements terminaux diméthylsilanol (Dimethiconol selon la dénomination CTFA) tels que les huiles de la série 48 de la société RHONE POULENC .

Dans cette classe de polyalkylsiloxanes, on peut également citer les produits vendus sous les dénominations "ABIL WAX 9800 et 9801" par la société GOLDSCHMIDT qui sont des polyalkyl (C₁-C₂₀) siloxanes.

Les polyalkylarylsiloxanes sont particulièrement choisis parmi les polydiméthyl mélhylphénylsiloxanes, les polydiméthyl diphénylsiloxanes linéaires et/ou ramifiés de viscosité de 1.10⁻⁵ à 5.10⁻²m²/s à 25°C.

Parmi ces polyalkylarylsiloxanes on peut citer à titre d'exemple les produits commercialisés sous les dénominations suivantes :
. les huiles SILBIONE de la série 70 641 de RHONE POULENC :
. les huiles des séries RHODORSIL 70 633 et 763 de RHONE POULENC ;
. l'huile DOW CORNING 556 COSMETIC GRAD FLUID de DOW CORNING ;
. les silicones de la série PK de BAYER comme le produit PK20 ;
. les silicones des séries PN, PH de BAYER comme les produits PN1000 et PH1000 ;
. certaines huiles des séries SF de GENERAL ELECTRIC telles que SF 1023, SF 1154, SF 1250, SF 1265.

Les gommes de silicone utilisables conformément à l'invention sont notamment des polydiorganosiloxanes ayant des masses moléculaires élevées comprises entre 200 000 et 1 000 000 utilisés seuls ou en mélange dans un solvant. Ce solvant peut être choisi parmi les silicones volatiles, les huiles polydiméthylsiloxanes (PDMS), les huiles polyphénylméthylsiloxanes (PPMS), les isoparaffines, les polyisobutylènes, le chlorure de méthylène, le pentane, le dodécane, le tridécanes ou leurs mélanges.

On peut plus particulièrement citer les produits suivants :
- polydiméthylsiloxane
- les gommes polydiméthylsiloxanes/méthylvinylsiloxane,
- polydiméthylsiloxane/diphénylméthylsiloxane,
- polydiméthylsiloxane/phénylméthylsiloxane,
- polydiméthylsiloxane/diphénylsiloxane/méthylvinylsiloxane.

Des produits plus particulièrement utilisables conformément à l'invention sont des mélanges tels que :
. les mélanges formés à partir d'un polydiméthylsiloxane hydroxylé en bout de chaîne (dénommé diméthiconol selon la nomenclature du dictionnaire CTFA) et d'un polydiméthylsiloxane cyclique (dénommé cyclométhicone selon la nomenclature du dictionnaire CTFA) tel que le produit Q2 1401 vendu par la société DOW CORNING ;
. les mélanges formés à partir d'une gomme polydiméthylsiloxane avec une silicone cyclique tel que le produit SF 1214 Silicone Fluid de la société GENERAL ELECTRIC, ce produit est une gomme SF 30 correspondant à une diméthicone, ayant un poids moléculaire de 500 000 solubilisée dans l'huile SF 1202 Silicone Fluid correspondant au décaméthylcyclopentasiloxane ;
. les mélanges de deux PDMS de viscosités différentes, et plus particulièrement d'une gomme PDMS et d'une huile PDMS, tels que le produit SF 1236 de la société GENERAL ELECTRIC. Le produit SF 1236 est le mélange d'une gomme SE 30 définie ci-dessus ayant une viscosité de 20 m²/s et d'une huile SF 96 d'une viscosité de 5.10⁻⁶m²/s, Ce produit comporte de préférence 15 % de gomme SE 30 et 85 % d'une huile SF 96.

Les résines d'organopolysiloxanes utilisables conformément à l'invention sont des systèmes siloxaniques réticulés renfermant les unités :
R₂SiO_{2/2}, R₃SiO_{1/2}, RSiO_{3/2} et SiO_{4/2} dans lesquelles R représente un groupement hydrocarboné possédant 1 à 16 atomes de carbone ou un groupement phényle. Parmi ces produits, ceux particulièrement préférés sont ceux dans lesquels R désigne un radical alkyle inférieur en C₁-C₄, plus particulièrement méthyle, ou un radical phényle.

On peut citer parmi ces résines le produit vendu sous la dénomination "DOW CORNING 593" ou ceux vendus sous les dénominations "SILICONE FLUID SS 4230 et SS 4267" par la société GENERAL ELECTRIC et qui sont des silicones de structure diméthyl/triméthyl siloxane.

On peut également citer les résines du type triméthylsiloxysilicate vendues notamment sous les dénominations X22-4914, X21-5034 et X21-5037 par la société SHIN-ETSU.

Les silicones organo modifiées utilisables conformément à l'invention sont des silicones telles que définies précédemment et comportant dans leur structure un ou plusieurs groupements organofonctionnels fixés par l'intermédiaire d'un radical hydrocarboné.

Parmi les silicones organomodifiées, on peut citer les polyorganosiloxanes comportant :
- des groupements polyéthylèneoxy et/ou polypropylèneoxy comportant éventuellement des groupements alkyle en C₆-C₂₄ tels que les produits dénommés diméthicone copolyol vendu par la société DOW CORNING sous la dénomination DC 1248 ou les huiles SILWET L 722, L 7500, L 77, L 711 de la société UNION CARBIDE et l'alkyl (C₁₂) méthicone copolyol vendue par la société DOW CORNING sous la dénomination Q2 5200 ;
- des groupements aminés substitués ou non comme les produits vendus sous la dénomination GP 4 Silicone Fluid et GP 7100 par la société GENESEE ou les produits vendus sous les dénominations Q2 8220 et DOW CORNING 929 ou 939 par la société DOW CORNING. Les groupements aminés substitués sont en particulier des groupements aminoalkyle en C₁-C₄ ;
- des groupements thiols comme les produits vendus sous les dénominations "GP 72 A" et "GP 71" de GENESEE ;
- des groupements alcoxylés comme le produit vendu sous la dénomination "SILICONE COPOLYMER F-755" par SWS SILICONES et ABIL WAX 2428, 2434 et 2440 par la société GOLDSCHMIDT ;
- des groupements hydroxylés comme les polyorganosiloxanes à fonction hydroxyalkyle décrits dans la demande de brevet français FR-A-85 16334 répondant à la formule (V) : 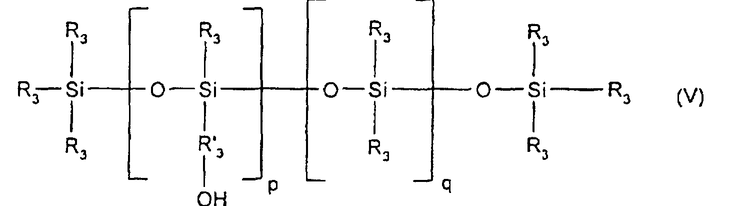 dans laquelle les radicaux R₃ identiques ou différents sont choisis parmi les radicaux méthyle et phényle ; au moins 60 % en mole des radicaux R₃ désignant méthyle ; le radical R'₃ est un chaînon alkylène divalent hydrocarboné en C₂-C₁₈ ; p est compris entre 1 et 30 inclus ; q est compris entre 1 et 150 inclus ;
- des groupements acyloxyalkyle tels que par exemple les polyorganosiloxanes décrits dans la demande de brevet français FR-A-2641185 et répondant à la formule (VI) : 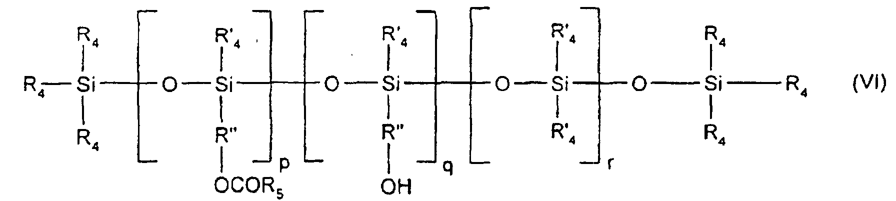 dans laquelle :
   R₄ désigne un groupement méthyle, phényle, -OCOR₅, hydroxyle, un seul des radicaux R₄ par atome de silicium pouvant être OH ;
   R'₄ désigne méthyle, phényle ; au moins 60 % en proportion molaire de l'ensemble des radicaux R₄ et R'₄ désignant méthyle ;
   R₅ désigne alkyle ou alcényle en C₈-C₂₀;
   R" désigne un radical alkylène hydrocarboné divalent, linéaire ou ramifié en C₂-C₁₈ ;
   r est compris entre 1 et 120 inclus ;
   p est compris entre 1 et 30 ;
   q est égal à 0 ou est inférieur à 0,5 p, p + q étant compris entre 1 et 30 ; les polyorganosiloxanes de formule (VI) peuvent contenir des groupements : 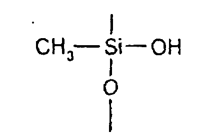 dans des proportions ne dépassant pas 15 % de la somme p + q + r.
      Les composés de formule (VI) peuvent être préparés par estérification de polyorganosiloxanes à fonction hydroxyalkyle de formule (V) ci-dessus.
   - des groupements anioniques du type carboxylique comme par exemple dans les produits décrits dans le brevet EP 186 507 de la société CHISSO CORPORATION, ou de type alkylcarboxyliques comme ceux présents dans le produit X-22-3701E de la société SHIN-ETSU ; 2-hydroxyalkylsulfonate; 2-hydroxyalkylthiosulfate tels que les produits vendus par la société GOLDSCHMIDT sous les dénominations "ABIL S201" et "ABIL S255".
   - des groupements hydroxyacylamino, comme les polyorganosiloxanes décrits dans la demande EP 342 834. On peut citer par exemple le produit Q2-8413 de la société DOW CORNING.

Selon l'invention, on peut également utiliser des silicones comprenant une portion polysiloxane et une portion constituée d'une chaîne organique non-siliconée, l'une des deux portions constituant la chaîne principale du polymère l'autre étant greffée sur la dite chaîne principale Ces polymères sont par exemple décrits dans les demandes de brevet EP-A-412 704, EP-A-412 707, EP-A-640 105 et WO 95100578, EP-A-582 152 et WO 93/23009 et les brevets US 4,693,935, US 4,728,571 et US 4,972,037. Ces polymères sont de préférence anioniques ou non ioniques.

De tels polymères sont par exemple les copolymères susceptibles d'être obtenus par polymérisation radicalaire à partir du mélange de monomères constitué par :
a) 50 à 90% en poids d'acrylate de tertiobutyle ;
b) 0 à 40% en poids d'acide acrylique ;
c) 5 à 40% en poids de macromère siliconé de formule : 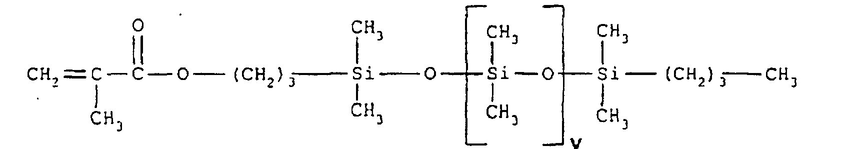 avec v étant un nombre allant de 5 à 700 ; les pourcentages en poids étant calculés par rapport au poids total des monomères.

D'autres exemples de polymères siliconés greffés sont notamment des polydiméthylsiloxanes (PDMS) sur lesquels sont greffés, par l'intermédiaire d'un chaînon de raccordement de type thiopropylène, des motifs polymères mixtes du type acide poly(méth)acrylique et du type poly(méth)acrylate d'alkyle et des polydiméthylsiloxanes (PDMS) sur lesquels sont greffés, par l'intermédiaire d'un chaînon de raccordement de type thiopropylène, des motifs polymères du type poly(méth)acrylate d'isobutyle.

Selon l'invention, toutes les silicones peuvent également être utilisées sous forme d'émulsions.

Les polyorganosiloxanes particulièrement préférés conformément à l'invention sont :
- les silicones non volatiles choisies dans la famille des polyalkylsiloxanes à groupements terminaux triméthylsilyle telles que les huiles ayant une viscosité comprise entre 0,2 et 2,5 m²/s à 25° C telles que les huiles de la séries DC200 de DOW CORNING en particulier celle de viscosité 60 000 c St. et 300 000 c St., des séries SILBIONE 70047 et 47 et plus particulièrement l'huile 70 047 V 500 000 commercialisées par la société RHONE POULENC, les polyalkylsiloxanes à groupements terminaux diméthylsilanol tels que les diméthiconol ou les polyalkylarylsiloxanes tels que l'huile SILBIONE 70641 V 200 commercialisée par la société RHONE POULENC ;
- les mélanges d'organopolysiloxanes et de silicones cycliques tels que le produit Q2 1401 commercialisé par la société DOW CORNING, et le produit SF 1214 commercialisé par la société GENERAL ELECTRIC ;
- les mélanges de deux PDMS de viscosités différentes notamment d'une gomme et d'une huile tels que le produit SF 1236 commercialisé par la société GENERAL ELECTRIC ;
- la résine d'organopolysiloxane commercialisée sous la dénomination DOW CORNING 593 ;
- les polysiloxane à groupements aminés tels que les amodiméthicone ou les triméthylsilylamodiméthicone ;

Les agents tensio-actifs utilisables dans les compositions moussantes de lavage et de conditionnement conformes à l'invention peuvent être choisis parmi les agents tensio-actifs anioniques, amphotères, zwittérioniques, non ioniques ou leurs mélanges ayant des propriétés détergentes et/ou moussantes.

Les tensioactifs convenant à la mise en oeuvre de la présente invention sont notamment les suivants :

### (i) Tensioactif(s) anionique(s) :

Ainsi, à titre d'exemple de tensio-actifs anioniques utilisables, seuls ou mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants les alkyisulfates, les alkyléthersulfates, alkylamidoéthersulfates, alkylarylpolyéthersulfates, mono-glycérides sulfates ; les alkylsulfonates, alkylphosphates, alkylamidosulfonates, alkyl-aryisulfonates, α-oléfine-sulfonates, paraffine-sulfonates ; les alkylsulfosuccinates, les alkyléthersulfosuccinates, les alkylamidosulfosuccinates; les alkylsulfosuccinamates ; les alkylsulfoacétates ; les alkylétherphosphates; les acylsarcosinates, les acyl-iséthionates et les N-acyltaurates, le radical alkyle ou acyle de tous ces différents composés comportant de préférence de 12 à 20 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, paimitique, stéanque, les acides d'huile de coprah ou d'huile de coprah hydrogénée ; les acyl-lactylates dont le radical acyle comporte 8 à 20 atomes de carbone. On peut également utiliser les alkylpolyglycosides comportant un groupement sulfate, sulfonate, succinate ou sulfosuccinate, les acides d'alkyl D galactoside uroniques et leurs sels ainsi que les acides éthers carboxyliques polyoxyalkylénés et leurs sels, en particulier ceux comportant de 2 à 50 groupements oxyde d'éthylène, et leurs mélanges. Les tensioactifs anioniques du type acides ou sels d'éthers carboxyliques polyoxyalkylénés sont en particulier ceux qui répondent à la formule (1) suivante : 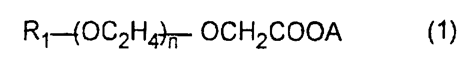 dans laquelle :
R₁ désigne un groupement alkyle ou alkylaryle, et n est un nombre entier ou décimal (valeur moyenne) pouvant varier de 2 à 24 et de préférence de 3 à 10, le radical alkyle ayant entre 6 et 20 atomes de carbone environ, et aryle désignant de préférence phényle,
A désigne H, ammonium, Na, K, Li, Mg ou un reste monoéthanolamine ou triéthanolamine. On peut également utiliser des mélanges de composés de formule (1) en particulier des mélanges dans lesquels les groupements R₁ sont différents.

Des composés de formule (1) sont vendus par exemple par la société CHEM Y sous les dénominations AKYPO (NP40, NP70, OP40, OP80, RLM25, RLM38, RLMQ 38 NV, RLM 45, RLM 45 NV, RLM 100, RLM 100 NV, RO 20, RO 90, RCS 60, RS 60, RS 100, RO 50) ou par la société SANDOZ sous les dénominations SANDOPAN (DTC Acid, DTC).

### (ii) Tensioactif(s) non ionique(s) :

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revêt pas, dans le cadre de la présente invention, de caractère critique. Ainsi, ils peuvent être notamment choisis parmi (liste non limitative) les alcools, les alpha-diols, les alkylphénols ou les acides gras polyéthoxylés, polypropoxylés ou polyglycérolés, ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkyl(C₈-C₂₀)polyglycosides éventuellement oxyalkylénés, les dérivés de N-alkyl glucamine, les oxydes d'amines tels que les oxydes d'aikyl (C₁₀-C₁₄) amines ou les oxydes de N-acylaminopropylmorpholine. On notera que les alkylpolyglycosides constituent des tensio-actifs non-ioniques rentrant particulièrement bien dans le cadre de la présente invention.

### (iii) Tensioactif(s) amphotère(s) ou zwittérionique(s) :

Les agents tensioactifs amphotères ou zwittérioniques, dont la nature ne revet pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 18 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) bétaïnes ou les alkyl (C₈-C₂₀) amidoalkyl (C₁-C₆) sulfobétaïnes.

Parmi les dérivés d'amines, on peut citer les produits vendus sous les dénomination MIRANOL, tels que décrits dans les brevets US-2 528 378 et US-2 781 354 et de structures :

R₂-CONHCH₂CH₂-N(R₃)(R₄)(CH₂COO-) (2)

dans laquelle : R₂ désigne un radical alkyle dérivé d'un acide R₂-COOH présent dans l'huile de coprah hydrolysée, un radical heptyle, nonyle ou undécyle, R₃ désigne un groupement bêta-hydroxyéthyle et R₄ un groupement carboxyméthyle ; et

R₂,-CONHCH₂CH₂-N(B)(C) (3)

dans laquelle :
B représente -CH₂CH₂OX', C représente -(CH₂)_{z} -Y', avec z = 1 ou 2,
X' désigne le groupement -CH₂CH₂-COOH ou un atome d'hydrogène
Y' désigne -COOH ou le radical -CH₂ - CHOH - SO3H
R₂, désigne un radical alkyle d'un acide R₉-COOH présent dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

Ces composés sont classés dans le dictionnaire CTFA, Sème édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Cocoamphodipropionic acid.
A titre d'exemple on peut citer le cocoamphodiacetate vendu sous la dénomination commerciale MIRANOL C2M concentré par la société RHONE POULENC.

Dans les compositions conformes à l'invention, on utilise de préférence des mélanges d'agents tensio-actifs et en particulier des mélanges d'agents tensio-actifs anioniques et d'agents tensio-actifs amphotères, zwittérioniques ou non ioniques. Un mélange particulièrement préféré est un mélange constitué d'au moins un agent tensio-actif anionique et d'au moins un agent tensio-actif amphotère ou zwittérionique.

On utilise de préférence un agent tensio-actif anionique choisi parmi les alkyl(C₁₂-C₁₄) sulfates de sodium, de triéthanolamine ou d'ammonium, les alkyl (C₁₂-C₁₄)éthersulfates de sodium oxyéthylénés à 2,2 moles d'oxyde d'éthylène, le cocoyl iséthionate de sodium et l'alphaoléfine(C₁₄-C₁₆) sulfonate de sodium et leurs mélange avec :
- soit un agent tensio-actif amphotère tel que les dérivés d'amine dénommés disodiumcocoamphodipropionate ou sodiumcocoamphopropionate vendus notamment par la société RHONE POULENC sous la dénomination commerciale "MIRANOL C2M CONC" en solution aqueuse à 38 % de matière active ou sous la dénomination MIRANOL C32;
- soit un agent tensio-actif zwittérionique tel que les alkylbétaïnes en particulier la cocoyibétaïne vendue sous la dénomination "DEHYTON AB 30" en solution aqueuse à 32 % de MA par la société HENKEL.

Le ou les dialkyléthers répondant à la formule (I) utilisés conformément à l'invention sont présents de préférence dans des proportions comprises entre 0,1 et 10 % par rapport au poids total de la composition et en particulier entre 0,5 et 5 %.

La ou les silicones peuvent être utilisées dans les compositions conformes à l'invention dans des proportions généralement comprises entre 0,05 et 20 %, et de préférence entre 0,1 et 10 % en poids par rapport au poids total de la composition.

Le ou les agents tensioactifs sont utilisés dans les compositions conformes à l'invention dans des proportions suffisantes pour conférer un caractère détergent à la composition, lesquelles sont comprises entre 5 et 50 % par rapport au poids total de la composition et en particulier entre 8 et 35 %.

Le pH de ces compositions est généralement compris entre 3 et 9 et plus particulièrement entre 4 et 8.

Le milieu aqueux peut être constitué uniquement par de l'eau ou par un mélange d'eau et d'un solvant cosmétiquement acceptable tel qu'un alcool inférieur en C₁-C₄, comme l'éthanol. l'isopropanol, le tertiobutanol, le n-butanol ; les alkylèneglycols comme le propylèneglycol, les éthers de glycols.

Les compositions conformes à l'invention peuvent contenir en plus de l'association définie ci-dessus des agents régulateurs de viscosité tels que des électrolytes, ou des agents épaississants. On peut citer en particulier le chlorure de sodium, le xylène sulfonate de sodium, les scléroglucanes, les gommes de xanthane, les alcanolamides d'acide gras, les alcanolamides d'acide alkyl éther carboxylique éventuellement oxyéthylénés avec jusqu'à 5 moles d'oxyde d'éthylène tel que le produit vendu sous la dénomination "AMINOL A15" par la société CHEM Y, les acides polyacryliques réticulés et les copolymères acide acrylique / acrylates d'alkyle en C₁₀-C₃₀ réticulés. Ces agents régulateurs de viscosité sont utilisés dans les compositions selon l'invention dans des proportions pouvant aller jusqu'à 10 % en poids par rapport au poids total de la composition.

Les compositions conformes à l'invention peuvent également contenir jusqu'à 3 % d'agents nacrants ou opacifiants bien connus dans l'état de la technique tels que par exemple les palmitates de sodium ou de magnésium, les stéarates et hydroxystéarates de sodium ou de magnésium, les dérivés acylés à chaîne grasse tels que les monostéarates ou distéarates d'éthylène glycol ou de polyéthylèneglycol.

Les compositions conformes à l'invention peuvent éventuellement contenir en outre d'autres agents ayant pour effet d'améliorer les propriétés cosmétiques de cheveux ou de la peau sans cependant altérer la stabilité des compositions. On peut citer à ce sujet les agents tensio-actifs cationiques, des polymères anioniques ou non ioniques ou cationiques ou amphotères, les protéines, les hydrolysats de protéines, les céramides, les pseudocéramides, les hydroxyacides, les vitamines, le panthénol, les huiles végétales, animales, minérales ou synthétiques.

Parmi les agents tensioactifs cationiques on peut citer en particulier (liste non limitative) : les sels d'amines grasses primaires, secondaires ou tertiaires, éventuellement polyoxyalkyiénées ; les sels d'ammonium quaternaire tels que les chlorures ou les bromures de tétraalkylammonium, d'alkylamidoalkyltrialkylammonium, de dialkyldihydroxyalkyl ammonium, de trialkylbenzylammonium, de trialkylhydroxyalkylammonium ou d'alkylpyridinium; les dérivés d'imidazoline, les diesters gras de diméthyl trihydroxyéthyl ammonium ; ou les oxydes d'amines à caractère cationique, les radicaux alkyles ayant de 1 à 4 atomes de carbone.

Les agents conditionneurs de type polymères cationiques utilisables conformément à la présente invention peuvent être choisis parmi tous ceux déjà connus en soi comme améliorant les propriétés cosmétiques des cheveux traités par des compositions détergentes, à savoir notamment ceux décrits dans la demande de brevet EP-A-0 337 354 et dans les demandes de brevets français FR-A-2 270 846, 2 383 660, 2 598 611, 2 470 596 et 2 519 863.

De manière encore plus générale, au sens de la présente invention, l'expression "polymère cationique" désigne tout polymère contenant des groupements cationiques et/ou des groupements ionisables en groupements cationiques.

Les polymères cationiques préférés sont choisis parmi ceux qui contiennent des motifs comportant des groupements amine primaires, secondaires, tertiaires et/ou quaternaires pouvant soit faire partie de la chaîne principale polymère, soit être portés par un substituant latéral directement relié à celle-ci.

Les polymères cationiques utilisés ont généralement une masse moléculaire en nombre comprise entre 500 et 5.10⁶ et de préférence comprise entre 10³ et 3.10⁶

Parmi les polymères cationiques, on peut citer plus particulièrement les protéines (ou hydrolysats de protéines) quaternisées et les polymères du type polyamine, polyaminoamide et polyammonium quaternaire. Ce sont des produits connus.

Les protéines ou hydrolysats de protéines quaternisés sont en particulier des polypeptides modifiés chimiquement portant en bout de chaîne, ou greffés sur celle-ci, des groupements ammonium quaternaire. Leur masse moléculaire peut varier par exemple de 1 500 à 10 000, et en particulier de 2 000 à 5 000 environ. Parmi ces composés, on peut citer notamment :
- les hydrolysats de collagène portant des groupements triéthylammonium tels que les produits vendus sous la dénomination "Quat-Pro E" par la société MAYBROOK et dénommés dans le dictionnaire CTFA "Triéthonium Hydrolyzed Collagen Ethosulfate" ;
- les hydrolysats de collagène portant des groupements chlorure de triméthylammonium et de triméthylstéarylammonium, vendus sous la dénomination de "Quat-Pro S" par la société Maybroook et dénommnés dans le dictionnaire CTFA "Steartrimonium Hydrolyzed Collagen" ;
- les hydrolysats de protéines animales portant des groupements triméthylbenzylammonium tels que les produits vendus sous la dénomination "CROTEIN BTA" par la société CRODA et dénommés dans le dictionnaire CTFA "Benzyltrimonium hydrolyzed animal protein" ;
- les hydrolysats de protéines portant sur la chaine polypeptidique des groupements ammonium quaternaire comportant au moins un radical alkyle ayant de 1 à 18 atomes de carbone.

Parmi ces hydrolysats de protéines, on peut citer entre autres :
- le "CROQUAT L" dont les groupements ammonium quaternaires comportent un groupement alkyle en C₁₂;
- le "CROQUAT M" dont les groupements ammonium quaternaires comportent des groupements alkyle en C₁₀-C₁₈ ;
- le "CROQUAT S" dont les groupements ammonium quaternaires comportent un groupement alkyle en C₁₈ ;
- le "CROTEIN Q" dont les groupements ammonium quaternaires comportent au moins un groupe alkyle ayant de 1 à 18 atomes de carbone.

Ces différents produits sont vendus par la société CRODA.

D'autre protéines ou hydrolysats quaternisés sont par exemple ceux répondant à la formule : 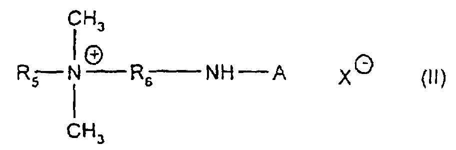 dans laquelle X⁻ est un anion d'un acide organique ou minéral, A désigne un reste de protéine dérivé d'hydrolysats de protéine de collagène, R₅ désigne un groupement lipophile comportant jusqu'à 30 atomes de carbone, R₆ représente un groupement alkylène ayant 1 à 6 atomes de carbone. On peut citer par exemple les produits vendus par la société Inolex, sous la dénomination "LEXEIN QX 3000", appelé dans le dictionnaire CTFA "Cocotrimonium Collagen Hydrolysate".

On peut encore citer les protéines végétales quaternisées telles que les protéines de blé, de maïs ou de soja : comme protéines de blé quaternisées, on peut citer celles commercialisées par la société CRODA sous les dénominations "Hydrotriticum WQ ou QM", appelées dans le dictionnaire CTFA "Cocodimonium Hydrolysed wheat protein", "Hydrotriticum QL" appelée dans le dictionnaire CTFA "Laurdimonium hydrolysed wheat protein", ou encore "Hydrotriticum QS", appelée dans le dictionnaire CTFA "Steardimonium hydrolysed wheat protein".

Les polymères du type polyamine, polyamidoamide, polyammonium quaternaire, utilisables conformément à la présente invention, pouvant être notamment mentionnés, sont ceux décrits dans les brevets français n° 2 505 348 ou 2 542 997. Parmi ces polymères, on peut citer :
(1) Les copolymères vinylpyrrolidone-acrylate ou -méthacrylate de dialkylamino-alkyle quaternisés ou non, tels que les produits vendus sous la dénomination "GAFQUAT" par la société ISP, comme par exemple GAFQUAT 734, 755 ou HS100 ou bien le produit dénommé "Copolymère 937". Ces polymères sont décrits en détail dans les brevets français 2 077 143 et 2 393 573.
(2) Les dérivés d'éthers de cellulose comportant des groupements ammonium quaternaires décrits dans le brevet français 1 492 597, et en particulier les polymères commercialisés sous les dénominations "JR" (JR 400, JR 125, JR 30M) ou "LR" (LR 400, LR 30M) par la société UNION CARBIDE Corporation. Ces polymères sont également définis dans le dictionnaire CTFA comme des ammonium quaternaires d'hydroxyéthylcellulose ayant réagi avec un époxyde substitué par un groupement triméthylammonium.
(3) Les dérivés de cellulose cationiques tels que les copolymères de cellulose ou les dérivés de cellulose greffés avec un monomère hydrosoluble d'ammonium quaternaire, et décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyléthyl triméthylammonium, de méthacryfmidopropyl triméthylammonium, de diallyl diméthylammonium.
   Les produits commercialisés répondant à cette définition sont plus particulièrement les produits vendus sous la dénomination "CELQUAT L 200" et "CELQUAT H 100" par la société NATIONAL STARCH.
(4) Les polysaccharides cationiques décrits plus particulièrement dans les brevets US 3 589 578 et 4 031 307 tel que les gommes de guar contenant des groupements cationiques trialkylammonium. On utilise par exemple des gommes de guar modifiées par un sel (par ex. chlorure) de 2,3-époxypropyl triméthylammonium.
   De tels produits sont commercialisés notamment sous les dénominations commerciales de JAGUAR C13 S, JAGUAR C 15, JAGUAR C 17 ou JAGUAR C162 par la société MEYHALL.
(5) les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361 ;
(6) les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyidiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polymaoamide ; ces polyaminoamides peuvent être alcoylés ou s'ils comportent une ou plusieurs fonctions amines tertiaires, quaternisées. De tels polymères sont notamment décrits dans les brevets français 2.252.840 et 2,368.508 ;
(7) les dérivés de polyaminoamides résultant de la condensation de polyalcoylènes polyamines avec des acides polycarboxyliques suivie d'une alcoylation par des agents bifonctionnels. On peut citer par exemple les polymères acide adipiquediacoyl-aminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363.
   Parmi ces dérivés, on peut citer plus particulièrement les polymères acide adipique/ diméthylaminohydroxypropyl/diéthylène triamine vendus sous la dénomination "CARTARETINE F, F4 ou F8" par la société SANDOZ.
(8) les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 :
   1; le polyaminoamide en résultant étant amené à réagir avec l'épichlorhydrine dans un rapport molaire d' épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.
   Des polymères de ce type sont en particulier commercialisés sous la dénomination "Hercosett 57" par la société Hercules Inc. ou bien sous la dénomination de "PD 170" ou "Delsette 101" par la société HERCULES dans le cas du copolymère d'acide adipique/époxypropyl/diéthylène-triamine,
(9) les cyclopolymères de méthyl diallyl amine ou de diallyl diméthyl ammonium tels que les homopolymères ou les copolymères comportant comme constituant principal de la chaîne des motifs répondant aux formules (VI) ou (VI') : 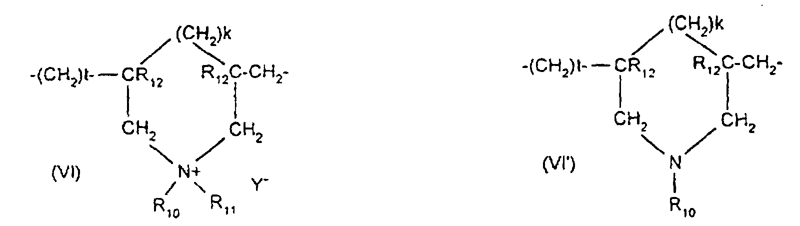 formules dans lesquelles k et t sont égaux à 0 ou 1, la somme k + t étant égale à 1 ; R₁₂ désigne un atome d'hydrogène ou un radical méthyle ; R₁₀ et R₁₁, indépendamment l'un de l'autre, désignent un groupement alkyle ayant de 1 à 22 atomes de carbone, un groupement hydroxyalkyle dans lequel le groupement alkyle a de préférence 1 à 5 atomes de carbone, un groupement amidoalkyle inférieur ou R₁₀ et R₁₁ peuvent désigner conjointement avec l'atome d'azote auquel ils sont rattachés, des groupement hétérocycliques, tels que pipéridinyle ou morpholinyle ; Y⁻ est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.
   Parmi les polymères définis ci-dessus, on peut citer plus particulièrement l'homopolymère de chlorure de diallyldiméthylammonium vendu sous la dénomination "MERQUAT 100" par la société MERCK.
(10) le polymère de diammonium quaternaire contenant des motifs récurants répondant à la formule : 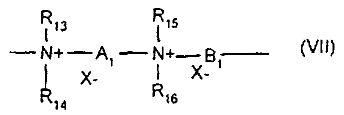 formule (VII) dans laquelle :
   R₁₃, R₁₄, R₁₅ et R₁₆, identiques ou différents, représentent des radicaux aliphatiques, alicycliques, ou arylaliphatiques contenant de 1 à 20 atomes de carbone ou des radicaux hydroxyalkylaliphatiques inférieurs, ou bien R₁₃, R₁₄, R₁₅ et R₁₆, ensemble ou séparément, constituent avec les atomes d'azote auxquels ils sont rattachés des hétérocycles contenant éventuellement un second hétéroatome autre que l'azote ou bien R₁₃, R₁₄, R₁₅ et R₁₆ représentent un radical alkyle en C₁-C₆ linéaire ou ramifié substitué par un groupement nitrile, ester, acyle, amide ou -CO-O-R₁₇-D ou -CO-NH-R₁₇-D où R₁₇ est un alkylène et D un groupement ammonium quaternaire ;
   A₁ et B₁ représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X⁻ désigne un anion dérivé d'un acide minéral ou organique;
   A₁, R₁₃ et R₁₅ peuvent former avec les deux atomes d'azote auxquels ils sont rattachés un cycle pipérazinique ; en outre si A₁ désigne un radical alkylène ou hydroxyalkylène linéaire ou ramifié, saturé ou insaturé, B₁ peut également désigner un groupement (CH₂)ₙ-CO-D-OC-(CH₂)ₙ-
   dans lequel D désigne:
   a) un reste de glycol de formule : -O-Z-O-, où Z désigne un radical hydrocarboné linéaire ou ramifié ou un groupement répondant à l'une des formules suivantes :

      -(CH₂-CH₂-O)ₓ-CH₂-CH₂-

      -[CH₂-CH(CH₃)-O]_{y}-CH₂-CH(CH₃)-

      où x et y désignent un nombre entier de 1 à 4, représentant un degré de polymérisation défini et unique ou un nombre quelconque de 1 à 4 représentant un degré de polymérisation moyen ;
   b) un reste de diamine bis-secondaire tel qu'un dérivé de pipérazine ;
   c) un reste de diamine bis-primaire de formule : -NH-Y-NH-, où Y désigne un radical hydrocarboné linéaire ou ramifié, ou bien le radical bivalent

      -CH₂₋CH₂-S-S-CH₂-CH₂- ;
   d) un groupement uréylène de formule : -NH-CO-NH- ;

   De préférence, X⁻ est un anion tel que le chlorure ou le bromure.
   Ces polymères ont une masse moléculaire en nombre généralement comprise entre 1000 et 100000.
   Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.
(11) les polymères de polyammonium quaternaires constitués de motifs de formule (VIII): 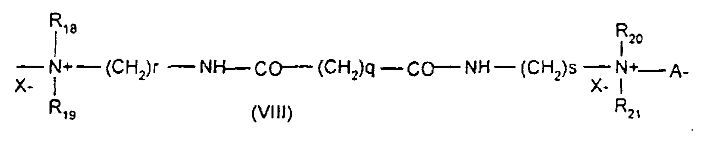 formule dans laquelle :
   R₁₈, R₁₉, R₂₀ et R₂₁, identiques ou différents, représentent un atome d'hydrogène ou un radical méthyle, éthyle, propyle, β-hydroxyéthyle, β-hydroxypropyle ou

      -CH₂CH₂(OCH₂CH₂)ₚOH,
   où p est égal à 0 ou à un nombre entier compris entre 1 et 6, sous réserve que R₁₈, R₁₉, R₂₀ et R₂₁ ne représentent pas simultanément un atome d'hydrogène,
   r et s, identiques ou différents, sont des nombres entiers compris entre 1 et 6,
   q est égal à 0 ou à un nombre entier compris entre 1 et 34,
   X désigne un atome d'halogène,
   A désigne un radical d'un dihalogénure ou représente de préférence

      -CH₂-CH₂-O-CH₂-CH₂-.

   De tels composés sont notamment décrits dans la demande de brevet EP-A-122 324.
   On peut par exemple citer parmi ceux-ci, les produits "MIRAPOL A 15", "MIRAPOL AD1", "MIRAPOL AZ1" et "MIRAPOL 175" vendus par la société MIRANOL.
(12) les homopolymères ou copolymères dérivés des acides acrylique ou méthacrylique et comportant des motifs : 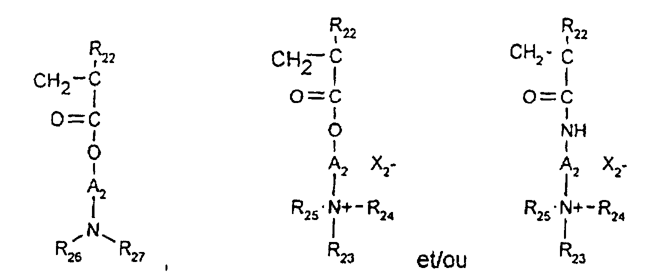
   dans lesquels les groupements R₂₂ désignent indépendamment H ou CH₃, les groupements A₁ désignent indépendamment un groupe alcoyle linéaire ou ramifié de 1 à 6 atomes de carbone ou un groupe hydroxyalcoyle de 1 à 4 atomes de carbone,
   les groupements R₂₃, R₂₄, R₂₅, identiques ou différents, désignant indépendamment un groupe alcoyle de 1 à 18 atomes de carbone ouun radical benzyle,
   les groupements R₂₆ et R₂₇ représentent unatome d'hydrogène ou un groupement alcoyle de 1 à 6 atomes de carbone,
   X₂⁻ désigne un anion, par exemple méthosulfate ou halogénure, tel que chlorure ou bromure.

   Le ou les comonomères utilisables dans la préparation des copolymères correspondants appartiennent à la famille des acrylamides, méthacrylamides, diacétone acrylamides, acrylamides et méthacrylamides substitués à l'azote par des alcoyle inférieurs, des esters d'alcoyles des acides acrylique ou méthacrylique, la vinylpyrrolidone ou des esters vinyliques.
(13) Les polymères quaternaires de vinylpyrrolidone et de vinylimidazole tels que par exemple les produits commercialisés sous les dénominations LUVIQUAT FC 905, FC 550 et FC 370 par la société B.A.S.F..
(14) Les polyamines comme le POLYQUART H vendu par HENKEL, référencé sous le nom de « POLYETHYLENEGLYCOL (15) TALLOW POLYAMINE » dans le dictionnaire CTFA.
(15) Les polymères réticulés de chlorure de méthacryloyloxyéthyltriméthylammonium tels que les polymères obtenus par homopolymérisation du diméthylaminoéthylméthacrylate quaternisé par le chlorure de méthyle, ou par copolymérisation de l'acryiamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloytoxyéthyl triméthylammonium (20/80 en poids) sous forme de dispersion contenant 50 % en poids dudit copolymère dans de l'huile minérale. Cette dispersion est commercialisée sous le nom de « SALCARE SC 92 » par la société ALLIED COLLOIDS. On peut également utiliser un homopolymère réticulé du chlorure de méthacryloyloxyéthyl triméthylammonium contenant environ 50 % en poids de l'homopolymère dans de l'huile minérale. Cette dispersion est commercialisée sous le nom de « SALCARE SC 95 » par la société ALLIED COLLOIDS.

D'autres polymères cationiques utilisables dans le cadre de l'invention sont des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Selon l'invention, on peut utiliser plus particulièrement les polymères choisis parmi le MIRAPOL, le composé de formule (VII) dans laquelle R₁₃, R₁₄, R₁₅ et R₁₆ représentent le radical méthyle, A₁ représente le radical de formule -(CH2)3- et B₁ représente le radical de formule -(CH₂)₆- et X⁻ représente l'anion chlorure (nommé ultérieurement Mexomère PO) et le composé de formule (VII) dans laquelle R₁₃ et R₁₄ représentent le radical éthyle, R₁₅ et R₁₆ représentent le radical méthyle, A₁ et B₁ représentent le radical de formule -(CH₂)₃- et X⁻ représente l'anion bromure (nommé ultérieurement Mexomère PAK).

Parmi tous les polymères cationiques susceptibles d'être utilisés dans le cadre de la présente invention, on préfère mettre en oeuvre les dérivés d'éther de cellulose quaternaires tels que les produits vendus sous la dénomination « JR 400 » par la société UNION CARBIDE CORPORATION, les cyclopolymères, en particulier les homopolymères du chlorure de diallyldiméthylammonium et les copolymères du chlorure de diallyldiméthylammonium et d'acrylamide, vendus sous les dénominations « MERQUAT 550 » et « MERQUAT S » par la société MERCK, les polysaccharides cationiques et plus particulièrement la gomme de guar modifiée par du chlorure de 2,3-époxypropyl triméthylammonium vendue sous la dénomination « JAGUAR C13S » par la société MEYHALL.

A titre de polymère amphotère, on peut citer:
- les polymères comportant de préférence environ 60 à environ 99 % de motifs dérivés d'un monomère de dialkyl diallyl ammonium, dans lequel les groupements alkyle comportent de 1 à 18 atomes de carbone et de préférence environ 1 à environ 40 % de motifs dérivés de monomères choisis parmi les acides acrylique et méthacrylique.
   Les polymères préférés sont les polymères de diallyldiméthyl- ou de diallyidiéthylammonium et d'acide acrylique tels que le produit commercialisé sous la dénomination MERQUAT 280 par la société MERCK ;
- les chitosane partiellement modifiés par des diacides carboxyliques en C₄-C₈ tels que ceux décrits dans le FR 2 137 684. Le taux de modification peut être compris entre 30 et 90% en poids par rapport au poids total du chitosane. Ces chitosane peuvent être totalement désacétylés.

Selon l'invention, le ou les polymères cationiques peuvent représenter de 0,001 % à 10 % en poids, de préférence de 0,005 % à 5 % en poids, et encore plus préférentiellement de 0,01 % à 3 % en poids, du poids total de la composition finale.

Les compositions selon l'invention peuvent contenir également des synergistes de mousses tels que des 1,2-alcanediols en C₁₀-C₁₈ ou des alcanolamides gras dérivés de mono ou de diéthanolamine.

Ces compositions peuvent également contenir différents adjuvants utilisés couramment en cosmétique tels que des parfums, des conservateurs, des agents séquestrants, des stabilisateurs de mousse et des agents acidifiants ou alcalinisants bien connus en cosmétique.

Les compositions selon l'invention sont utilisés de préférence comme shampooings pour le lavage et le conditionnement des cheveux et ils sont appliqués dans ce cas-là sur le cheveux humides dans des quantités efficaces pour les laver, cette application étant suivie d'un rinçage à l'eau, après un éventuel temps de pose.

Les compositions conformes à l'invention sont également utilisables comme gels douche pour le lavage et le conditionnement des cheveux et de la peau, auquel cas ils sont appliqués sur la peau et les cheveux humides et sont rincés après application.

Les exemples suivants sont destinés à illustrer l'invention :

### EXEMPLE 1

On a réalisé une composition de shampooing contenant :
- Lauryléthersulfate de sodium (C12/C14 à 70/30) à 2,2 moles d'oxyde d'éthylène (MA = matière active) 14 gMA
- Tensioactif amphotère dérivé d'imidazoline vendu sous la dénomination MIRANOL C2M par la société RHONE POULENC à 40% de MA 4 gMA
- Cétostéaryl (50/50 en poids) sulfate de sodium 0,75 g
- Gomme de guar modifiée par du chlorure de 2,3-époxypropyl triméthylammonium vendue sous la dénomination JAGUAR C13 S par la société RHONE POULENC 0,2 g
- Polydiméthylsiloxane vendu sous la dénomination de MIRASIL DM 500 000 par la société RHONE POULENC 2,6 g
- Distéaryléther 4 g
- Alcool cétylstéarylique 1 g
- Alcool stéarylique oxyéthyléné à 10 moles d'oxyde d'éthylène (BRIJ 76 de ICI) 0,8 g
- Acide citrique qs pH 5
- Parfum, conservateurs qs
- Eau déminéralisée qsp 100 g

La composition est stable et présente un bon effet nacré.

On effectue un shampooing en appliquant environ 12 g de la composition sur des cheveux préalablement mouillés. On fait mousser le shampooing puis on rince abondamment à l'eau.
Les cheveux se peignent facilement, sont brillants et présentent un toucher doux.
Le pouvoir moussant de la composition est supérieur à 100 ml.

### EXEMPLE 2

On a réalisé une composition de shampooing contenant :
- Lauryléthersulfate de sodium (C12/C14 à 70/30) à 2,2 moles d'oxyde d'éthylène (MA = matière active) 14 gMA
- Cocoyl bétaine en solution aqueuse à 32% de matière active (DEHYTON AB 30 de HENKEL) 2,56 gMA
- Cétostéaryl (50/50 en poids) sulfate de sodium 0,75 g
- Polydiméthylsiloxane de viscosité 60 000 cSt vendu par la société DOW CORNING sous la dénomination FLUID DC 200 - 60 000 cSt 1,5 g
- Amodiméthicone vendue en émulsion cationique à 35% de matière active sous la dénomination FLUID DC 939 par la société DOW CORNING 1 gMA
- Distéaryléther 1,5 g
- Distéarate d'éthylèneglycol 1 g
- Monoisopropanolamide d'acides de coprah 2 g
- Acide citrique qs pH 5
- Parfum, conservateurs qs
- Eau déminéralisée qsp 100 g

La composition est stable et présente un bon effet nacré.
On effectue un shampooing en appliquant environ 12 g de la composition sur des cheveux préalablement mouillés. On fait mousser le shampooing puis on rince abondamment à l'eau.
Les cheveux se peignent facilement, sont brillants et présentent un toucher doux.
Le pouvoir moussant de la composition est supérieur à 100 ml.

### EXEMPLE 3

On a réalisé une composition de shampooing contenant :
- Lauryléthersulfate de sodium (C12/C14 à 70/30) à 2,2 moles d'oxyde d'éthylène (MA = matière active) 14 gMA
- Tensioactif amphotère dérivé d'imidazoline vendu sous la dénomination MIRANOL C2M par la société RHONE POULENC à 40% de MA 4 gMA
- Cétostéaryl (50/50 en poids) sulfate de sodium 0,75 g
- Gomme de guar modifiée par du chlorure de 2,3-époxypropyl triméthylammonium vendue sous la dénomination JAGUAR C13 S parla société RHONE POULENC 0,2 g
- Polydiméthylsiloxane de viscosité 60 000 cSt vendu par la société DOW CORNING sous la dénomination FLUID DC 200 - 60 000 cSt 2,6 g
- Distéaryléther 2,5 g
- Mélange d'alcool décylique, laurique et palmitique oxyéthyléné (85/8,5/6,5 en poids) vendu sous la dénomination MERGITAL BL 309 par la société HENKEL 1,5 g
- Acide citrique qs pH 5
- Parfum, conservateurs qs
- Eau déminéralisée qsp 100 g

La composition est stable et présente un bon effet nacré.
On effectue un shampooing en appliquant environ 12 g de la composition sur des cheveux préalablement mouillés. On fait mousser le shampooing puis on rince abondamment à l'eau.
Les cheveux se peignent facilement, sont brillants et présentent un toucher doux.
Le pouvoir moussant de la composition est supérieur à 100 ml.

### EXEMPLE 4

On a réalisé une composition de shampooing contenant :
- Lauryléthersulfate de sodium (C12/C14 à 70/30) à 2,2 moles d'oxyde d'éthylène (MA = matière active) 14 gMA
- Cocoyl bétaïne en solution aqueuse à 32% de matière active (DEHYTON AB 30 de HENKEL) 2,56 gMA
- Cétostéaryl (50/50 en poids) sulfate de sodium 0,75 g
- Polydiméthylsiloxane α-ω-hydroxy en émulsion aqueuse à 51% de MA vendu par la société OSI sous la dénomination TP512 1,5 gMA
- Distéaryléther 2,5 g
- Distéarate d'éthylèneglycol 1 g
- Monoisopropanolamide d'acides de coprah 2 g
- Acide citrique qs pH 5
- Parfum, conservateurs qs
- Eau déminéralisée qsp 100 g

Cette composition présentent les mêmes propriétés que celles de la composition de l'exemple 1.

## Revendications

1. Composition moussante de lavage et de conditionnement des matières kératiniques, en particulier des cheveux et de la peau, caractérisée par le fait qu'elle comprend, dans un milieu aqueux cosmétiquement acceptable, au moins une silicone, au moins un agent tensio-actif possédant des propriétés détergentes et au moins un dialkyléther solide à température inférieure ou égale à 30°C, de formule (I) :
R-O-R' (I)
dans laquelle :
R et R', identiques ou différents, désignent un radical alkyle, saturé ou insaturé, linéaire ou ramifié, comportant de 12 à 30 atomes de carbone, le pouvoir moussant de la composition étant supérieur à 50 ml,
les agents tensio-actifs étant présents dans des proportions comprises entre 5 et 50 % en poids par rapport au poids total de là composition.

2. Composition selon la revendication 1, caractérisée par le fait que R et R' sont identiques.

3. Composition selon la revendication 2, caractérisée par le fait que R et R' désignent un radical stéaryle.

4. Composition selon l'une quelconque des revendications 1 à 3, caractérisée par le fait que les silicones sont choisies parmi les polyorganosiloxanes insolubles dans la composition et se présentent sous forme d'huiles, de cires, de résines ou de gommes.

5. Composition selon l'une quelconque des revendications 1 à 4, caractérisée par le fait que les polyorganosiloxanes sont des polyorganosiloxanes non volatils choisis parmi les polyalkylsiloxanes, les polyarylsiloxanes, les polyalkylarylsiloxanes, les gommes et résines de silicones, les polyorganosiloxanes modifiés par des groupements organofonctionnels ainsi que leurs mélanges.

6. Composition selon l'une quelconque des revendications 1 à 5, caractérisée par le fait que :
(a) les polyalkylsiloxanes sont choisis parmi :
- les polydiméthylsiloxanes à groupements terminaux triméthylsilyle ;
- les polydiméthylsiloxanes à groupements terminaux diméthylsilanol ;
- les polyalkyl(C₁-C₂₀)siloxanes ;
(b) les polyalkylarylsiloxanes sont choisis parmi :
- les polydiméthylméthylphénylsiloxanes, les polydiméthyldiphényl siloxanes linéaires et/ou ramifiés de viscosité comprise entre 1.10⁻⁵ et 5.10⁻²m²/s à 25°C ;
(c) les gommes de silicone sont choisies parmi les polydiorganosiloxanes ayant des masses moléculaires comprises entre 200 000 et 1 000 000 utilisés seuls ou sous forme de mélange dans un solvant ;
(d) les résines sont choisies parmi les résines constituées d'unités : R₃ Si O_{1/2}, R₂ Si O_{2/2}, R Si O_{3/2}, Si O_{4/2}
dans lesquelles R représente un groupement hydrocarboné ayant de 1 à 16 atomes de carbone ou un groupement phényle ;
(e) les silicones organo modifiées sont choisies parmi les silicones comportant dans leur structure un ou plusieurs groupements organofonctionnels fixés par l'intermédiaire d'un radical hydrocarboné.

7. Composition selon la revendication 6, caractérisée par le fait que les gommes de silicone utilisées seules ou sous forme de mélange sont choisies parmi les structures suivantes :
- polydiméthylsiloxane
- polydiméthylsiloxane/méthylvinylsiloxanes,
- polydiméthylsiloxaneldiphénylsiloxane,
- polydiméthylsiloxane/phénylméthylsiloxane,
- polydiméthylsiloxane/diphénylsiloxane/méthylvinylsiloxanes et les mélanges suivants:
- des mélanges formés à partir d'un polydiméthylsiloxane hydroxylé en bout de chaîne et d'un polydiméthylsiloxane cyclique ;
- les mélanges formés à partir d'une gomme polydiméthylsiloxane et d'une silicone cyclique ; et
- des mélanges de polydiméthylsiloxanes de viscosités différentes.

8. Composition selon la revendication 6, caractérisée par le fait que les silicones organomodifiées sont choisies parmi les polyorganosiloxanes comportant :
a) des groupements polyéthylèneoxy et/ou polypropylèneoxy ;
b) des groupements aminés substitués ou non ;
c) des groupements thiols ;
d) des groupements alcoxylés ;
e) des groupements hvdroxvalkvle répondant à la formule suivante : 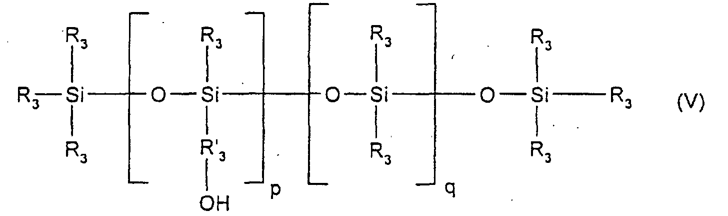 dans laquelle les radicaux R₃ identiques ou différents sont choisis parmi les radicaux méthyle et phényle, au moins 60 % en mole des radicaux R₃ représentant méthyle ; le radical R'₃ est un chaînon alkylène divalent hydrocarboné en C₂-C₁₈; p est compris entre 1 et 30 inclus ; q est compris entre 1 et 150 inclus ;
f) des groupements acyloxyalkyle répondant à la formule suivante : 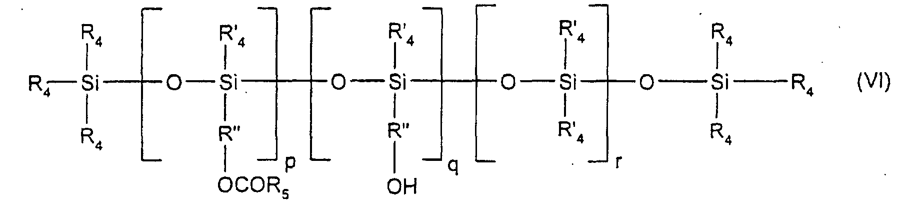 dans laquelle :
R₄ désigne méthyle, phényle, -OCOR₅, hydroxyle, un seul des radicaux R₄ par atome de silicium pouvant être OH ;
R'₄ désigne méthyle, phényle ; au moins 60 % en mole de l'ensemble des radicaux R₄ et R'₄ désignant méthyle ;
R₅ désigne alkyle ou alcényle en C₈-C₂₀ ;
R" désigne un radical alkylène hydrocarboné divalent, linéaire ou ramifié en C₂-C₁₈ ;
r est compris entre 1 et 120 inclus ;
p est compris entre 1 et 30 ;
q est égal à 0 ou est inférieur à 0,5 p, p + q étant compris entre 1 et 30 ; les polyorganosiloxanes de formule (VI) pouvant contenir des groupements ; 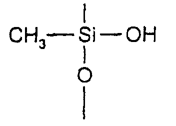 dans des proportions ne dépassant pas 15 % de la somme p + q + r ;
g) des groupements alkyl carboxyliques,
h) des groupements 2-hydroxyalkylsulfonates,
i) des groupements 2-hydroxyalkylthiosulfonates,
j) des groupements hydroxyacylamino.

9. Composition selon l'une quelconque des revendications 1 à 8, caractérisée par le fait que les polyorganosiloxanes sont choisis parmi les polyalkylsiloxanes à groupements terminaux triméthylsilyle, les polyalkylsiloxanes à groupements terminaux diméthylsilanol, les polyalkylarylsiloxanes, les mélanges de deux PDMS constitués d'une gomme et d'une huile de viscosités différentes, les mélanges d'organosiloxanes et de silicones cycliques, les résines d'organopolysiloxanes.

10. Composition selon l'une quelconque des revendications 1 à 4, caractérisée par le fait que les silicones sont choisies parmi les silicones volatiles.

11. Composition selon la revendication 10, caractérisée par le fait que les silicones volatiles sont choisies parmi :
- les silicones cycliques comportant de 3 à 7 atomes de silicium ;
- les cyclopolymères du type diméthylsiloxanes/méthylalkylsiloxane de structure : 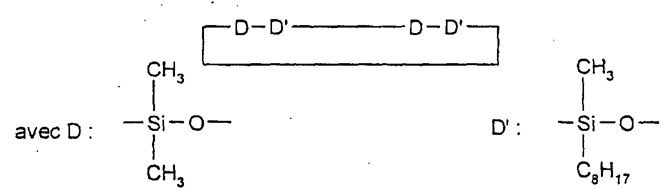
- les mélanges de silicones cycliques avec des composés organiques dérivés du silicium ;
- les silicones volatiles linéaires ayant 2 à 9 atomes de silicium et de viscosité inférieure ou égale à 5.10⁻⁶m²/s à 25° C.

12. Composition selon l'une quelconque des revendications 1 à 11, caractérisée par le fait que les agents tensio-actifs détergents sont choisis parmi les agents tensio-actifs anioniques, amphotères, zwittérioniques, non ioniques ou leurs mélanges.

13. Composition selon l'une quelconque des revendications 1 à 12, caractérisée par le fait que l'on utilise des mélanges de tensio-actifs choisis parmi les mélanges de tensio-actifs anioniques et d'agents tensio-actifs amphotères, zwittérioniques ou non ioniques.

14. Composition selon l'une quelconque des revendications 1 à 13, caractérisée par le fait que la ou les silicones sont utilisées dans les compositions conformes à l'invention dans des proportions comprises entre 0,05 et 20 % et de préférence entre 0,1 et 10 % en poids par rapport au poids total de la composition.

15. Composition selon l'une quelconque des revendications 1 à 14, caractérisée par le fait que les dialkyléther de formule (I) sont présents dans des proportions comprises entre 0,1 et 10 % en poids par rapport au poids total de la composition et en particulier entre 0,5 et 5 %.

16. Composition selon l'une quelconque des revendications 1 à 15, caractérisée par le fait que les agents tensio-actifs sont présents dans des proportions comprises entre 8 et 35 % en poids par rapport au total de la composition.

17. Composition selon l'une quelconque des revendications 1 à 16, caractérisée par le fait que le pH est compris entre 3 et 9 et en particulier 3 et 8.

18. Composition selon l'une quelconque des revendications 1 à 17, caractérisée par le fait que le milieu aqueux est constitué par de l'eau ou par un mélange d'eau et d'un solvant cosmétiquement acceptable choisi parmi les alcools inférieurs en C₁-C₄ les alkylèneglycols et les éthers de glycol.

19. Composition selon l'une quelconque des revendications 1 à 18, caractérisée par le fait que la composition contient en plus des agents régulateurs de viscosité choisis parmi les électrolytes, ou des agents épaississants présents en des proportions pouvant aller jusqu'à 10 % en poids par rapport au poids total de la composition.

20. Composition selon l'une quelconque des revendications 1 à 19, caractérisée par le fait qu'elle contient en plus jusqu'à 3 % d'agents nacrants et/ou opacifiants.

21. Composition selon l'une quelconque des revendications 1 à 20, caractérisée par le fait qu'elle contient en plus un ou plusieurs adjuvants destinés à améliorer les propriétés cosmétiques choisis par les tensio-actifs cationiques, les polymères anioniques ou non ioniques ou cationiques ou amphotères ou des protéines éventuellement quaternisées.

22. Composition selon la revendication 21, caractérisée par le fait que les polymères cationiques sont choisis parmi les polymères comportant des groupements aminés primaires, secondaires, tertiaires et/ou quaternaires faisant partie de la chaîne polymère ou directement reliés à celle-ci et ayant un poids moléculaire de 500 à 5 000 000.

23. Composition selon la revendication 22, caractérisée par le fait que les polymères cationiques sont choisis parmi les dérivés d'éther de cellulose quaternaires, les cyclopolymères, les polysaccharides cationiques et leurs mélanges.

24. Composition selon la revendication 23, caractérisée par le fait que ledit cyclopolymère est choisi parmi les homopolymères du chlorure de diallyidiméthylammonium et les copolymères du chlorure de diallyidiméthylammonium et d'acrylamide.

25. Composition selon la revendication 23, caractérisée par le fait que lesdits dérivés d'éther de cellulose quaternaires sont choisis parmi les hydroxyéthylcelluloses ayant réagi avec un époxyde substitué par un groupement triméthylammonium.

26. Composition selon la revendication 23, caractérisée par le fait que lesdits polysaccharides cationiques sont choisis parmi les gommes de guar modifiées par un sel de 2,3-époxypropyl triméthylammonium.

27. Composition selon l'une quelconque des revendications 1 à 26, caractérisée par le fait par le fait que le polymère amphotère est un copolymère de dialkyl diallyl ammonium et d'acide acrylique ou méthacrylique et/ou un chitosane partiellement modifié par un diacide carboxylique.

28. Composition selon l'une quelconque des revendications 1 à 27, caractérisée par le fait qu'elle contient différents adjuvants cosmétiquement acceptables choisis parmi les parfums, les conservateurs, les séquestrants, les synergistes de mousses, les stabilisateurs de mousses, les agents acidifiants ou alcalinisants.

29. Utilisation comme shampooing de la composition telle que définie dans l'une quelconque des revendications 1 à 28.

30. Utilisation comme gel douche de la composition telle que définie dans l'une quelconque des revendications 1 à 28.

31. Procédé de lavage et de conditionnement des matières kératiniques, caractérisé par le fait que l'on applique sur ces matières au moins une composition telle que définie dans l'une quelconque des revendications 1 à 28, et qu'après un éventuel temps de pose on rince à l'eau les matières traitées.

32. Utilisation d'un dialkyléther gras tel que défini à l'une des revendications 1 à 3, comme agent de mise en suspension d'une silicone dans une composition moussante de lavage et conditionnement contenant, dans un milieu aqueux cosmétiquement acceptable, des tensioactifs.

## Patentansprüche

1. Schaumbildende Zusammensetzung zum Waschen und Konditionieren von Keratinmaterialien, insbesondere der Haare und der Haut,
dadurch gekennzeichnet, daß sie in einem kosmetisch akzeptablen Medium mindestens ein Silicon, mindestens einen grenzflächenaktiven Stoff, der reinigende Eigenschaften aufweist, und mindestens einen Dialkylether, der bei einer Temperatur von 30 °C oder darunter fest ist, der Formel
R-O-R' (I)
enthält, in der bedeuten:
R und R', die gleich oder verschieden sind, gesättigte oder ungesättigte, geradkettige oder verzweigte Alkylreste, die 12 bis 30 Kohlenstoffatome enthalten, wobei das Schaumbildungsvermögen größer als 50 ml ist und wobei die grenzflächenaktiven Stoffen in einem Mengenanteil von 5 bis 50 Gew.-%, bezogen auf das Gesamtgewicht der Zusammensetzung, enthalten sind.

2. Zusammensetzung nach Anspruch 1, dadurch gekennzeichnet, daß R und R' identisch sind.

3. Zusammensetzung nach Anspruch 1, dadurch gekennzeichnet, daß es sich bei R und R' um einen Stearylrest handelt.

4. Zusammensetzung nach einem der Ansprüche 1 bis 3, dadurch gekennzeichnet, daß die Silicone unter den Polyorganosiloxanen ausgewählt sind, die in der Zusammensetzung unlöslich sind und in Form von Ölen, Wachsen, Harzen oder Gummis vorliegen.

5. Zusammensetzung nach einem der Ansprüche 1 bis 4, dadurch gekennzeichnet, daß es sich bei den Polyorganosiloxanen um nicht flüchtige Polyorganosiloxane handelt, die unter den Polyalkylsiloxanen, Polyarylsiloxanen, Polyalkylarylsiloxanen, den Silicongummis und Siliconharzen, den Polyorganosiloxanen, die mit funktionellen organischen Gruppen modifiziert sind, und den Gemischen dieser Polyorganosiloxane ausgewählt sind.

6. Zusammensetzung nach einem der Ansprüche 1 bis 5, dadurch gekennzeichnet, daß
(a) die Polyalkylsiloxane ausgewählt sind unter
- den Polydimethylsiloxanen mit endständigen Trimethylsilyl-Gruppen,
- den Polydimethylsiloxanen mit endständigen Dimethylsilanol-Gruppen,
- den Poly-(C₁-C₂₀)-alkylsiloxanen,
(b) die Polyalkylarylsiloxane ausgewählt sind unter
- den geradkettigen und/oder verzweigten Polydimethylmethylphenylsiloxanen und Polydimethyldiphenylsiloxanen, die bei 25 °C eine Viskosität im Bereich von 1·10⁻⁵ bis 5·10⁻² m²/s aufweisen.
(c) den Silicongummis, die unter den Polydiorganosiloxanen ausgewählt sind, die eine Molmasse im Bereich von 200000 bis 1000000 aufweisen, die einzeln oder in Form eines Gemischs in einem Lösemittel verwendet werden,
(d) die Siliconharze unter den Harzen ausgewählt sind, die aus Einheiten R₃SiO_{1/2}, R₂SiO_{2/2}, RSiO_{3/2}, SiO_{4/2} aufgebaut sind, worin R eine Kohlenwasserstoffgruppe, die 1 bis 16 Kohlenstoffatome aufweist, oder eine Phenylgruppe darstellt,
(e) die organisch modifizierten Silicone unter den Siliconen ausgewählt sind, die in ihrer Struktur eine oder mehrere funktionelle organische Gruppen enthalten, die über einen Kohlenwasserstoffrest angebunden sind.

7. Zusammensetzung nach Anspruch 6, dadurch gekennzeichnet, daß die Silicongummis, die einzeln oder in Form eines Gemischs verwendet werden, ausgewählt sind unter den folgenden Strukturen:
- Polydimethylsiloxan,
- Polydimethylsiloxan/Methylvinylsiloxane,
- Polydimethylsiloxan/Diphenylsiloxan,
- Polydimethylsiloxan/Phenylmethylsiloxan,
- Polydimethylsiloxan/Diphenylsiloxan/Methylvinylsiloxane,
und den folgenden Gemischen:
- den Gemischen, die aus einem Polydimethylsiloxan, das am Kettenende hydroxyliert ist, und einem cyclischen Polydimethylsiloxan gebildet sind,
- den Gemischen, die aus einem Polydimethylsiloxangummi und einem cyclischen Silicion gebildet sind, und
- den Gemischen von Polydimethylsiloxanen mit unterschiedlicher Viskosität.

8. Zusammensetzung nach Anspruch 6, dadurch gekennzeichnet, daß die organisch modifizierten Silicone unter den Polyorganosiloxanen ausgewählt sind, die enthalten:
a) Polyethylenoxy- und/oder Polypropylenoxy-Gruppen,
b) Amingruppen, die gegebenenfalls substituiert sind,
c) Thiolgruppen,
d) Alkoxygruppen,
e) Hydroxyalkylgruppen, die der folgenden Formel: 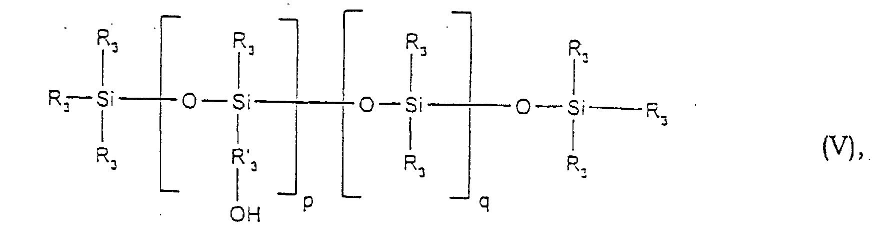 entsprechen, in der bedeuten:
- die Reste R₃ Reste, die gleich oder verschieden sind und unter Methyl und Phenyl ausgewählt sind, wobei mindestens 60 Mol-% der Reste R₃ Methyl darstellen,
- der Rest R'₃ eine zweiwertige C₂-C₁₈-Kohlenwasserstoffalkylenkette,
- p eine Zahl im Bereich von 1 bis 30, wobei die Grenzen eingeschlossen sind,
- q eine Zahl im Bereich von 1 bis 150, wobei die Grenzen eingeschlossen sind,
f) Acyloxyalkylgruppen, die der folgenden Formel 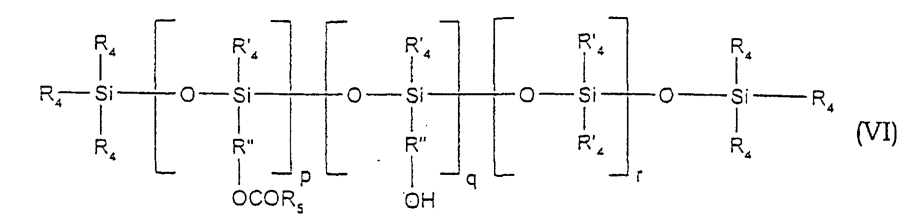 entsprechen, in der bedeuten:
- R₄ Methyl, Phenyl, -OCOR₅, Hydroxy, wobei pro Siliciumatom nur einer der Reste R₄ eine Hydroxygruppe sein kann,
- R'₄ Methyl, Phenyl, wobei mindestens 60 Mol-% aller Reste R₄ und R'₄ Methyl bedeuten,
- R₅ Alkyl oder Alkenyl mit 8 bis 20 Kohlenstoffatomen,
- R" eine zweiwertige, geradkettige oder verzweigte C₂-C₁₈-Kohlenwas serstoffalkylenkette,
- r eine Zahl im Bereich von 1 bis 120, wobei die Grenzen eingeschlossen sind,
- p eine Zahl im Bereich von 1 bis 30,
- q die Zahl 0 oder eine Zahl kleiner als 0,5 p, wobei p + q im Bereich von 1 bis 30 liegen,
wobei die Polysiloxane der Formel (VI) Gruppen 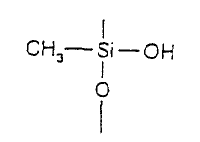 in einem Anteil enthalten können, der nicht mehr als 15 % der Summe aus p + q + r entspricht,
g) Alkylcarboxy-Gruppen,
h) 2-Hydroxyalkylsulfonat-Gruppen,
i) 2-Hydroxyalkylthiosulfonat-Gruppen,
j) Hydroxyacylamino-Gruppen.

9. Zusammensetzung nach einem der Ansprüche 1 bis 8, dadurch gekennzeichnet, daß die Polyorganosiloxangruppen unter den Polyalkylsiloxanen mit endständigen Trimethylsilyl-Gruppen, den Polyalkylsiloxanen mit endständigen Dimethylsilanol-Gruppen, den Polyalkylarylsiloxanen, den Gemischen aus zwei PDMS, die aus einem Gummi und einem Öl mit unterschiedlicher Viskosität bestehen, den Gemischen von Organopolysiloxanen und cyclischen Siliconen, den Organopolysiloxan-Harzen ausgewählt sind.

10. Zusammensetzung nach einem der Ansprüche 1 bis 4, dadurch gekennzeichnet, daß die Silicone unter den flüchtigen Siliconen ausgewählt sind.

11. Zusammensetzung nach Anspruch 10, dadurch gekennzeichnet, daß die flüchtigen Silicone ausgewählt sind unter:
- den cyclischen Siliconen, die 3 bis 7 Siliciumatome enthalten,
- den Cyclopolymeren vom Dimethylsiloxan/Methylalkyl-Siloxan-Typ der Struktur 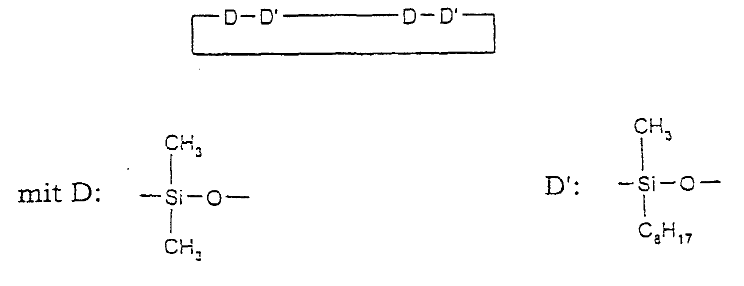
- den Gemischen cyclischer Silicone mit organischen, von Silicium abgeleiteten Verbindungen,
- den flüchtigen geradkettigen Siliconen, die 2 bis 9 Siliciumatome aufweisen, mit einer Viskosität bei 25 °C von 5.10⁻⁶ m²/s oder darunter.

12. Zusammensetzung nach einem der Ansprüche 1 bis 11, dadurch gekennzeichnet, daß die reinigenden grenzflächenaktiven Stoffe unter den anionischen, amphoteren, zwitterionischen, nichtionischen grenzflächenaktiven Stoffen und ihren Gemischen ausgewählt sind.

13. Zusammensetzung nach einem der Ansprüche 1 bis 12, dadurch gekennzeichnet, daß Gemische grenzflächenaktiver Stoffe verwendet werden, die unter den Gemischen anionischer grenzflächenaktiver Stoffe und amphoterer, zwitterionischer oder nichtionischer grenzflächenaktiver Stoffe ausgewählt sind.

14. Zusammensetzung nach einem der Ansprüche 1 bis 13, dadurch gekennzeichnet, daß das oder die Silicone in den erfindungsgemäßen Zusammensetzungen in einem Mengenanteil von 0,05 bis 20 Gew.-%, vorzugsweise 0,1 bis 10 Gew.-%, bezogen auf das Gesamtgewicht der Zusammensetzung, verwendet werden.

15. Zusammensetzung nach einem der Ansprüche 1 bis 14, dadurch gekennzeichnet, daß die Dialkylether der Formel (I) in einem Mengenanteil von 0,1 bis 10 Gew.-%, bezogen auf das Gesamtgewicht der Zusammensetzung, und insbesondere in einem Mengenanteil von 0,5 bis 5 Gew.-% enthalten sind.

16. Zusammensetzung nach einem der Ansprüche 1 bis 15, dadurch gekennzeichnet, daß die grenzflächenaktiven Stoffe in einem Mengenanteil von 8 bis 35 Gew.-%, bezogen auf das Gesamtgewicht der Zusammensetzung, enthalten sind.

17. Zusammensetzung nach einem der Ansprüche 1 bis 16, dadurch gekennzeichnet, daß der pH-Wert im Bereich von 3 bis 9 und vorzugsweise 3 bis 8 liegt.

18. Zusammensetzung nach einem der Ansprüche 1 bis 17, dadurch gekennzeichnet, daß das wäßrige Medium aus Wasser oder einem Gemisch aus Wasser und einem kosmetisch akzeptablen Lösemittel besteht, das unter den niederen C₁-C₄-Alkoholen, den Alkylenglykolen und den Glykolethern ausgewählt ist.

19. Zusammensetzung nach einem der Ansprüche 1 bis 18, dadurch gekennzeichnet, daß die Zusammensetzung außerdem Mittel zur Regulierung der Viskosität, die unter den Elektrolyten ausgewählt sind, oder Verdickungsmittel enthält, die in einem Mengenanteil enthalten sind, der bis zu 10 Gew.-%, bezogen auf das Gesamtgewicht der Zusammensetzung, betragen kann.

20. Zusammensetzung nach einem der Ansprüche 1 bis 19, dadurch gekennzeichnet, daß sie außerdem bis zu 3 % Perlglanzmittel oder Trübungsmittel enthält.

21. Zusammensetzung nach einem der Ansprüche 1 bis 20, dadurch gekennzeichnet, daß sie außerdem einen oder mehrere Hilfsstoffe enthält, die dafür vorgesehen sind, die kosmetischen Eigenschaften zu verbessern, die unter kationischen grenzflächenaktiven Stoffen, anionischen, nichtionischen, kationischen oder amphoteren Polymeren und gegebenenfalls quaternisierten Proteinen ausgewählt sind.

22. Zusammensetzung nach Anspruch 21, dadurch gekennzeichnet, daß die kationischen Polymere unter den Polymeren ausgewählt sind, die primäre, sekundäre, tertiäre und/oder quartäre Amingruppen enthalten, die Teil der Polymerkette sind oder unmittelbar mit der Polymerkette verbunden sind, und ein Molekulargewicht von 500 bis 5000000 aufweisen.

23. Zusammensetzung nach Anspruch 22, dadurch gekennzeichnet, daß die kationischen Polymere unter den quartären Derivaten von Celluloseethern, den Cyclopolymeren, den kationischen Polysacchariden und ihren Gemischen ausgewählt sind.

24. Zusammensetzung nach Anspruch 23, dadurch gekennzeichnet, daß das Cyclopolymer unter den Homopolymeren des Diallyldimethylammoniumchlorids und den Copolymeren aus Diallyldimethylammoniumchlorid und Acrylamid ausgewählt ist.

25. Zusammensetzung nach Anspruch 23, dadurch gekennzeichnet, daß die quartären Derivate von Celluloseethern unter den Hydroxyethylcellulosen ausgewählt sind, die mit einem Epoxid, das mit einer Trimethylammoniumgruppe substituiert ist, umgesetzt wurden.

26. Zusammensetzung nach Anspruch 23, dadurch gekennzeichnet, daß die kationischen Polysaccharide unter den mit einem 2,3-Epoxypropyltrimethylammoniumsalz modifizierten Guargummen ausgewählt sind.

27. Zusammensetzung nach einem der Ansprüche 1 bis 26, dadurch gekennzeichnet, daß das amphotere Polymer ein Copolymer aus Dialkylallylammonium und Acrylsäure oder Methacrylsäure und/oder einem Chitosan, das teilweise mit einer Dicarbonsäure modifiziert ist, ist.

28. Zusammensetzung nach einem der Ansprüche 1 bis 27, dadurch gekennzeichnet, daß sie verschiedene kosmetisch akzeptable Hilfsstoffe enthält, die unter Parfüms, Konservierungsmitteln, Maskierungsmitteln, die Schaumbildung synergistisch verbessernden Mitteln, Schaumstabilisatoren, Säuerungsmitteln und alkalisch machenden Mitteln ausgewählt sind.

29. Verwendung der wie in einem der Ansprüche 1 bis 28 definierten Zusammensetzung als Haarwaschmittel.

30. Verwendung der wie in einem der Ansprüche 1 bis 28 definierten Zusammensetzung als Duschgel.

31. Verfahren zum Waschen und Konditionieren von Keratinmaterialien, dadurch gekennzeichnet, daß auf die Keratinmaterialien mindestens eine wie in einem der Ansprüche 1 bis 28 definierte Zusammensetzung aufgetragen wird und daß die behandelten Keratinmaterialien, gegebenenfalls nach einer Einwirkungszeit der Zusammensetzung, ausgespült werden.

32. Verwendung eines Fettdialkylethers, der wie in einem der Ansprüche 1 bis 3 definiert ist, als Mittel zum Suspendieren eines Silicons in einer schaumbildenden Zusammensetzung zum Waschen und Konditionieren, die in einem kosmetisch akzeptablen Medium grenzflächenaktive Stoffe enthält.

## Claims

1. Foaming composition for washing and conditioning keratin substances, in particular the hair and the skin, characterized in that it comprises, in a cosmetically acceptable aqueous medium, at least one silicone, at least one surfactant with detergent properties and at least one dialkyl ether which is solid at a temperature of less than or equal to 30°C, of formula (I):
R-O-R' (I)
in which:
R and R', which may be identical or different, denote a linear or branched, saturated or unsaturated alkyl radical containing from 12 to 30 carbon atoms, the foaming power of the composition being greater than 50 ml, the surfactants being present in proportions of between 5% and 50% by weight relative to the total weight of the composition.

2. Composition according to Claim 1, characterized in that R and R' are identical.

3. Composition according to Claim 2, characterized in that R and R' denote a stearyl radical.

4. Composition according to any one of Claims 1 to 3, characterized in that the silicones are chosen from polyorganosiloxanes that are insoluble in the composition and are in the form of oils, waxes, resins or gums.

5. Composition according to any one of Claims 1 to 4, characterized in that the polyorganosiloxanes are non-volatile polyorganosiloxanes chosen from polyalkylsiloxanes, polyarylsiloxanes, polyalkylarylsiloxanes, silicone gums and resins and polyorganosiloxanes modified with organofunctional groups, and mixtures thereof.

6. Composition according to any one of Claims 1 to 5, characterized in that:
(a) the polyalkylsiloxanes are chosen from:
- polydimethylsiloxanes containing trimethylsilyl end groups;
- polydimethylsiloxanes containing dimethylsilanol end groups;
- poly (C₁-C₂₀) alkylsiloxanes ;
(b) the polyalkylarylsiloxanes are chosen from:
- linear and/or branched polydimethylmethylphenylsiloxanes and polydimethyldiphenylsiloxanes with a viscosity of between 1 × 10⁻⁵ and 5 × 10⁻² m²/s at 25°C;
(c) the silicone gums are chosen from polydiorganosiloxanes with molecular masses of between 200 000 and 1 000 000, used alone or in the form of a mixture in a solvent;
(d) the resins are chosen from resins consisting of units:
R₃SiO_{1/2}, R₂SiO_{2/2}, RSiO_{3/2}, SiO_{4/2} in which R represents a hydrocarbon-based group containing from 1 to 16 carbon atoms or a phenyl group;
(e) the organomodified silicones are chosen from silicones containing in their structure one or more organofunctional groups attached via a hydrocarbon-based radical.

7. Composition according to Claim 6, characterized in that the silicone gums used, alone or in the form of a mixture, are chosen from the following structures:
- polydimethylsiloxane
- polydimethylsiloxane/methylvinylsiloxanes,
- polydimethylsiloxane/diphenylsiloxane,
- polydimethylsiloxane/phenylmethylsiloxane,
- polydimethylsiloxane/diphenylsiloxane/methylvinylsiloxanes and the following mixtures:
- mixtures formed from a polydimethylsiloxane hydroxylated at the end of the chain and from a cyclic polydimethylsiloxane;
- mixtures formed from a polydimethylsiloxane gum and from a cyclic silicone; and
- mixtures of polydimethylsiloxanes of different viscosities.

8. Composition according to Claim 6, characterized in that the organomodified silicones are chosen from polyorganosiloxanes containing:
a) polyethylenoxy and/or polypropylenoxy groups;
b) substituted or unsubstituted amine groups;
c) thiol groups;
d) alkoxylated groups;
e) hydroxyalkyl groups corresponding to the following formula: 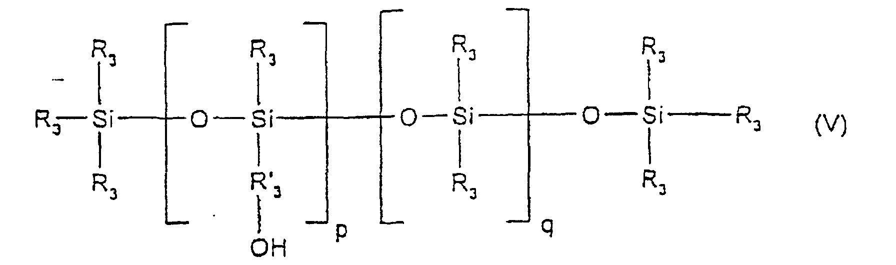 in which the radicals R₃, which may be identical or different, are chosen from methyl and phenyl radicals, at least 60 mol% of the radicals R₃ denoting methyl; the radical R'₃ is a divalent C₂-C₁₈ hydrocarbon-based alkylene chain unit; p is between 1 and 30 inclusive; q is between 1 and 150 inclusive;
f) acyloxyalkyl groups corresponding to the following formula: 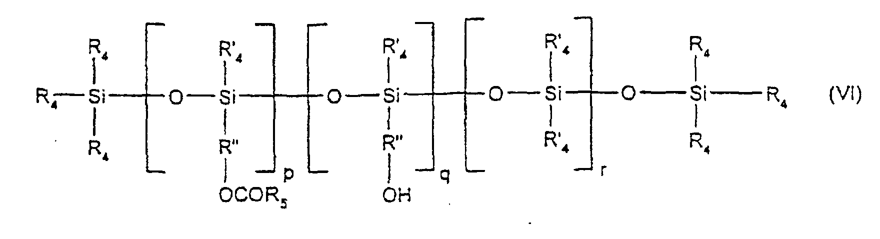 in which:
R₄ denotes methyl, phenyl, -OCOR₅ or hydroxyl, only one of which radicals R₄ per silicon atom may be OH;
R'₄ denotes methyl, phenyl; at least 60 mol% of all of the radicals R₄ and R'₄ denoting methyl;
R₅ denotes C₈-C₂₀ alkyl or alkenyl;
R" denotes a linear or branched, divalent C₂-C₁₈ hydrocarbon-based alkylene radical;
r is between 1 and 120 inclusive;
p is between 1 and 30;
q is equal to 0 or is less than 0.5 p, p + q being between 1 and 30; the polyorganosiloxanes of formula (VI) can contain groups: 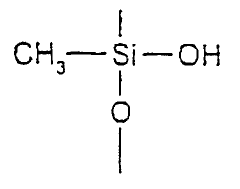 in proportions not exceeding 15% of the sum p + q + r;
g) alkylcarboxylic groups,
h) 2-hydroxyalkyl sulphonate groups,
i) 2-hydroxyalkyl thiosulphonate groups,
j) hydroxyacylamino groups.

9. Composition according to any one of Claims 1 to 8, characterized in that the polyorganosiloxanes are chosen from polyalkylsiloxanes containing trimethylsilyl end groups, polyalkylsiloxanes containing dimethylsilanol end groups, polyalkylarylsiloxanes, mixtures of two PDMSs consisting of a gum and an oil of different viscosities, mixtures of organosiloxanes and of cyclic silicones, and polyorganosiloxane resins.

10. Composition according to any one of Claims 1 to 4, characterized in that the silicones are chosen from volatile silicones.

11. Composition according to Claim 10, characterized in that the volatile silicones are chosen from:
- cyclic silicones containing from 3 to 7 silicon atoms;
- cyclopolymers of the dimethylsiloxanes/ methylalkylsiloxane type, with the structure: 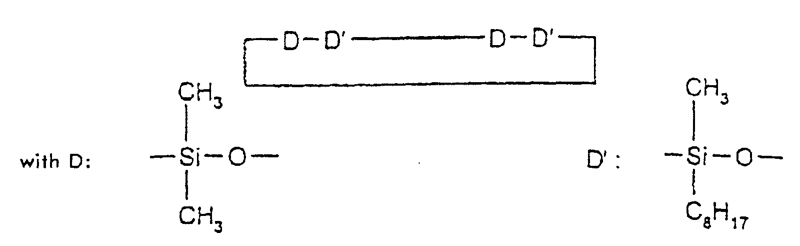
- mixtures of cyclic silicones with organosilicon compounds;
- linear volatile silicones containing from 2 to 9 silicon atoms and with a viscosity of less than or equal to 5 × 10⁻⁶ m²/s at 25°C.

12. Composition according to any one of Claims 1 to 11, characterized in that the detergent surfactants are chosen from anionic, amphoteric, zwitterionic and nonionic surfactants, or mixtures thereof.

13. Composition according to any one of Claims 1 to 12, characterized in that mixtures of surfactants chosen from mixtures of anionic surfactants with amphoteric, zwitterionic or nonionic surfactants are used.

14. Composition according to any one of Claims 1 to 13, characterized in that the silicone(s) is (are) used in the compositions in accordance with the invention in proportions of between 0.05 and 20%, and preferably between 0.1 and 10%, by weight, relative to the total weight of the composition.

15. Composition according to any one of Claims 1 to 14, characterized in that the dialkyl ethers of formula (I) are present in proportions of between 0.1 and 10% by weight relative to the total weight of the composition, and in particular between 0.5 and 5%.

16. Composition according to any one of Claims 1 to 15, characterized in that the surfactants are present in proportions of between 8 and 35% by weight relative to the total weight of the composition.

17. Composition according to any one of Claims 1 to 16, characterized in that the pH is between 3 and 9 and in particular between 3 and 8.

18. Composition according to any one of Claims 1 to 17, characterized in that the aqueous medium consists of water or of a mixture of water and a cosmetically acceptable solvent chosen from C₁-C₄ lower alcohols, alkylene glycols and glycol ethers.

19. Composition according to any one of Claims 1 to 18, characterized in that the composition also contains viscosity modifiers chosen from electrolytes, or thickeners present in proportions which can range up to 10% by weight relative to the total weight of the composition.

20. Composition according to any one of Claims 1 to 19, characterized in that it also contains up to 3% of pearlescent agents and/or opacifiers.

21. Composition according to any one of Claims 1 to 20, characterized in that it also contains one or more adjuvants intended to improve the cosmetic properties, chosen from cationic surfactants, anionic or nonionic or cationic or amphoteric polymers, or proteins, which are optionally quaternized.

22. Composition according to Claim 21, characterized in that the cationic polymers are chosen from polymers containing primary, secondary, tertiary and/or quaternary amine groups forming part of the polymer chain or directly connected thereto and having a molecular weight from 500 to 5 000 000.

23. Composition according to Claim 22, characterized in that the cationic polymers are chosen from quaternary cellulose ether derivatives, cyclopolymers and cationic polysaccharides, and mixtures thereof.

24. Composition according to Claim 23, characterized in that the said cyclopolymer is chosen from dimethyldiallylammonium chloride homopolymers and copolymers of dimethyldiallylammonium chloride and of acrylamide.

25. Composition according to Claim 23, characterized in that the said quaternary cellulose ether derivatives are chosen from hydroxyethylcelluloses which have reacted with an epoxide substituted with a trimethylammonium group.

26. Composition according to Claim 23, characterized in that the said cationic polysaccharides are chosen from guar gums modified with a 2,3-epoxypropyltrimethylammonium salt.

27. Composition according to any one of Claims 1 to 26, characterized in that the amphoteric polymer is a copolymer of dialkyldiallylammonium and of acrylic or methacrylic acid and/or a chitosan partially modified with a dicarboxylic acid.

28. Composition according to any one of Claims 1 to 27, characterized in that it contains various cosmetically acceptable adjuvants chosen from fragrances, preserving agents, sequestering agents, foam synergists, foam stabilizers and acidifying or basifying agents.

29. Use, as a shampoo, of the composition as defined in any one of Claims 1 to 28.

30. Use, as a shower gel, of the composition as defined in any one of Claims 1 to 28.

31. Process for washing and conditioning keratin substances, characterized in that at least one composition as defined in any one of Claims 1 to 28 is applied to these substances and, after an optional period of leaving the composition to stand on the substances, the treated substances are rinsed with water.

32. Use of a fatty dialkyl ether as defined in any one of Claims 1 to 3, as an agent for suspending a silicone in a washing and conditioning foaming composition containing surfactants in a cosmetically acceptable aqueous medium.
